(19) Europäisches Patentamt
European Patent Office
Office européen des brevets

(11) Veröffentlichungsnummer: **0 022 208**
**B1**

# (12) EUROPÄISCHE PATENTSCHRIFT

(45) Veröffentlichungstag der Patentschrift: **16.06.82**

(51) Int. Cl.³: **C 07 C 69/74**, C 07 C 67/313

(21) Anmeldenummer: **80103522.1**

(22) Anmeldetag: **24.06.80**

(54) **Verfahren zur Herstellung von 2-Formyl-3,3-dimethyl-cyclopropan-1-carbonsäureestern.**

(30) Priorität: **05.07.79 DE 2927133**

(43) Veröffentlichungstag der Anmeldung: **14.01.81 Patentblatt 81/2**

(45) Bekanntmachung des Hinweises auf die Patenterteilung: **16.06.82 Patentblatt 82/24**

(84) Benannte Vertragsstaaten: **BE CH DE FR GB IT LI NL**

(56) Entgegenhaltungen:
**DE-A1-2 615 160**
**Chemical Abstracts Band 76, Nr. 11**
**13. März 1972**
**Columbus, Ohio, USA**
**T. Van ES et al. "Aldehydes from aromatic nitriles. p-Formylbenzene-sulfonamide"**
**Seite 430, Spalte 1, Abstract Nr. 59 164d**

(73) Patentinhaber: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Priesnitz, Uwe, Dr., Heinrich-Heine Strasse 42, D-4750 Unna-Massen (DE)**
Erfinder: **Riebel, Hans-Jochem, Dr., In der Beek 92, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von 2-Formyl-3,3-dimethyl-cyclopropan-1-carbonsäureestern

Die Erfindung betrifft ein neues Verfahren zur Herstellung von bekannten 2-Formyl-3,3-dimethyl-cyclopropan-1-carbonsäureestern, welche als Zwischenprodukte zur Herstellung von Schädlingsbekämpfungsmiteln verwendet werden können.

Es ist bereits bekannt, dass man 2-Formyl-3,3-dimethyl-cyclopropan-1-carbonsäure-alkylester erhält, wenn man Chrysanthemumsäureester mit Ozon behandelt (vgl. US-PS 3679667). Die Verwendung von Ozon ist jedoch wegen der Toxizität dieser Verbindung und der Neigung von Ozonisierungsprodukten, sich explosionsartig zu zersetzen, mit hohen Risiken behaftet.

Weiter ist bekannt, dass man 2-Formyl-3,3-dimethyl-cyclopropan-1-carbonsäure-alkylester erhält, wenn man Acetale von 4-Oxo-2-butensäure-alkylestern mit Triphenyl-isopropyl-phosphoniumhalogeniden in Gegenwart von starken Basen umsetzt (vgl. DE-OS 2615160). Da die hierbei zu verwendenden starken Basen, wie z.B. Butyllithium, sehr empfindlich gegen Luftsauerstoff und Feuchtigkeit sind, müssen die Umsetzungen unter einer Inertgasatmosphäre und unter Verwendung sorgfältig getrockneter Lösungsmittel durchgeführt werden. Weitere Nachteile sind die hohen Kosten der Reagentien und die unbefriedigenden Ausbeuten bei dieser Synthesemethode.

Es ist eine Methode zur Reduktion von Nitrilen zu Aldehyden mit Ameisensäure an Raney-Nickel bekannt geworden, welche einfach durchzuführen ist und die Synthese von Aldehyden in guten Ausbeuten ermöglicht (vgl. J. Chem. Soc. [London] 1964, 5580-5581). Die Anwendung dieser Reaktionsmethode ist jedoch bisher auf eine geringe Zahl von aromatischen Nitrilen, welche keine weiteren, der Cyanogruppe vergleichbaren, funktionellen Gruppen aufweisen, beschränkt.

Es wurde nun ein Verfahren zur Herstellung von 2-Formyl-3,3-dimethyl-cyclopropan-1-carbonsäureestern der Formel I

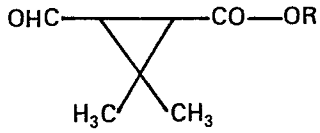

(I)

in welcher R für Alkyl oder für gegebenenfalls substituiertes Aralkyl steht, gefunden, welches dadurch gekennzeichnet ist, dass man 2-Cyano-3,3-dimethyl-cyclopropan-1-carbonsäureester der Formel II

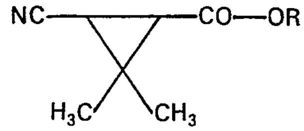

(II)

in welcher R die oben angegebene Bedeutung hat, mit Ameisensäure in Gegenwart von Katalysatoren, die zur katalytischen Hydrierung dienen, bei Temperaturen zwischen 0 und 150°C umsetzt.

Es ist als überraschend zu bezeichnen, dass nach dem erfindungsgemässen Verfahren 2-Formyl- 3,3-dimethyl-cyclopropan- 1-carbonsäureester aus 2-Cyano-3,3-dimethyl-cyclopropan-1-carbonsäureestern in sehr guten Ausbeuten und in hoher Reinheit hergestellt werden können, da damit zu rechnen war, dass auch die Esterkomponente und der Cyclopropan-Ring mit dem Reduktionsmittel reagieren würden.

Das neue Verfahren ist wesentlich einfacher und kostengünstiger als die bekannten Verfahren zur Herstellung von 2-Formyl-3,3-dimethyl-cyclopropan-carbonsäureestern durchzuführen.

Verwendet man als Ausgangsverbindung beispielsweise 2-Cyano-3,3-dimethyl-cyclopropan-1-carbonsäure-isopropylester, so kann die Reaktion dieser Verbindung mit Ameisensäure, beispielsweise unter Verwendung von Raney-Nickel als Katalysator, durch folgendes Formelschema skizziert werden:

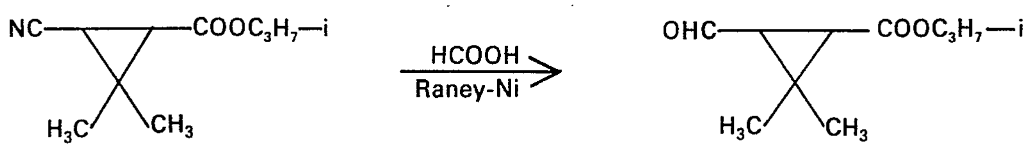


Die als Ausgangsverbindungen zu verwendenden 2-Cyano-3,3-dimethyl-cyclopropan-1-carbonsäureester sind durch die Formel (II) definiert. Vorzugsweise steht darin R für Alkyl mit 1 bis 4 Kohlenstoffatomen.

Als Beispiele seien genannt:

2-Cyano-3,3-dimethyl-cyclopropan-1-carbonsäure-methylester, -äthylester, -n-propylester, -isopropylester, -n-butylester, -iso-butylester, -sek.-butylester und tert.-butylester.

Ausgangsverbindungen der Formel (II) sind bekannt (vgl. BE-PS 858138, Bull. Soc. Chim. Belg. 87 [1978], 721-732). Soweit die Ausgangsverbindungen neu sind, können sie nach im Prinzip bekannten Verfahren hergestellt werden, beispielsweise, indem man 3,3-Dimethyl-cyclopropan-1,1,2-cyano-dicarbonsäure-diester mit etwa der äquimolaren Menge Wasser in einem polaren Lösungsmittel, wie z.B. 1-Oxo-1-methyl-phospholin, gegebenenfalls in Gegenwart eines Alkalihalogenides, wie z.B. Natriumbromid, bei Temperaturen zwischen 150 und 200°C umsetzt. Die Aufarbeitung kann auf übliche Weise durchgeführt werden, beispielsweise indem man mit Wasser verdünnt, mit einem mit Wasser nicht mischbaren Lösungsmittel, wie z.B. toluol extrahiert, die Extrakte trocknet und destilliert. 3,3-Dimethyl-cyclopropan-1,1,2-cyano-dicarbonsäureester, wie z.B. 3,3-Dimethyl-cyclopropan-2-cyano-1,1-dicarbon-säure-dimethylester, 3,3-Dimethyl-cyclopropan-2-cyano-1,1-dicarbonsäure-diäthylester und 3,3-Dimethyl-cyclopropan-1-cyano-1,2-dicarbonsäure-diäthylester sind bekannt [vgl. Bull. Soc. Chim. Belg. 86 (1977), 55-63; ibid 87 (1978), 721-732; J. Org. Chem. 32 (1967), 3351-3355].

Das erfindungsgemässe Verfahren wird in Gegenwart eines Katalysators durchgeführt. Es können die üblicherweise bei katalytischen Hydrierungen eingesetzten Metallkatalysatoren verwendet werden. Als bevorzugter Katalysator sei Raney-Nickel genannt.

Die Temperatur wird zwischen 0 und 150°C, vorzugsweise zwischen 50 und 100°C gehalten. Das Verfahren wird bei Normaldruck oder bei geringfügig erhöhtem oder vermindertem Druck durchgeführt.

Ameisensäure wird beim erfindungsgemässen Verfahren als Reaktand und als Verdünnungsmittel verwendet und dementsprechend in hohem Überschuss, vorzugsweise in einer Menge zwischen 10 und 50 Mol je Mol Ausgangsverbindung der Formel (II) eingesetzt.

Zur Durchführung des erfindungsgemässen Verfahrens wird das Gemisch aus 3,3-Dimethyl-2-cyano-cyclopropan-1-carbonsäureester (II), Ameisensäure und Raney-Nickel-Katalysator etwa eine Stunde bei der oben angegebenen Temperatur gerührt.

Das Reaktionsgemisch wird dann auf Raumtemperatur abgekühlt und mit wässrigem Alkohol verdünnt. Nach Filtration wird das Filtrat mit Wasser versetzt und mit einem mit Wasser nicht mischbaren Lösungsmittel, wie z.B. Chloroform extrahiert. Die nichtwässrige Phase wird getrocknet, filtriert und destilliert. Zur Charakterisierung der Produkte dient die Siedetemperatur.

Die nach dem erfindungsgemässen Verfahren herzustellenden 2-Formyl-3,3-dimethyl-cyclopropan-carbonsäureester können als Zwischenprodukte zur Herstellung von insektizid und akarizid wirksamen Pyrethroiden verwendet werden (vgl. DE-OS 2326077).

Beispiel

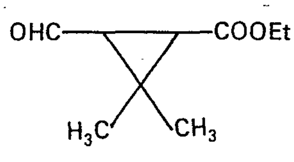

Eine Suspension von 10 g feuchtem Raney-Ni-Katalysator und 16,7 g (0,1 Mol) 2-Cyano-3,3-dimethyl-cyclopropan-1-carbonsäure-äthylester in 150 ml Ameisensäure wird 1 Stunde auf 80°C erhitzt. Dann wird das Reatkionsgemisch abgekühlt und mit 200 ml Äthanol/Wasser Gemisch (2/1) versetzt. Es wird filtriert. Das Filtrat wird mit 200 ml Wasser versetzt und dann zweimal mit je 100 ml Chloroform extrahiert. Die Chloroformlösung wird über $Na_2SO_4$ getrocknet und eingeengt. Der Rückstand wird fraktioniert. Man erhält 15 g (88% der Theorie) 2-Formyl-3,3-dimethyl-cyclopropan-1-carbonsäure-äthylester in Form eines farblosen Öls vom Siedepunkt Kp 75-77°/1 mm Hg.

In analoger Weise können hergestellt werden

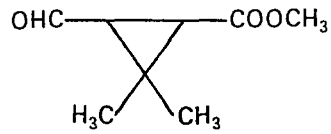

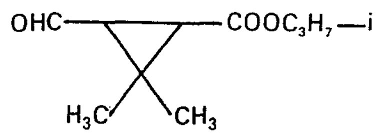

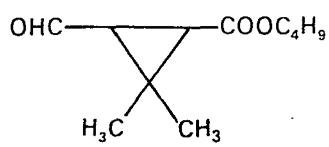

**Patentanspruch**

Verfahren zur Herstellung von 2-Formyl-3,3-dimethyl-cyclopropan-1-carbonsäureestern der Formel I

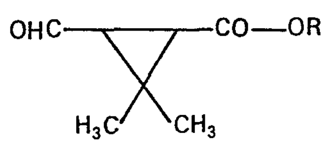

in welcher R für Alkyl oder für gegebenenfalls substituiertes Aralkyl steht, dadurch gekennzeichnet, dass man 2-Cyano-3,3-dimethyl-cyclopropan-1-carbonsäureester der Formel II

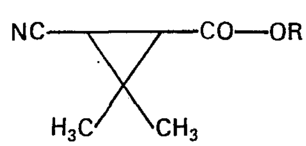

in welcher R die oben angegebene Bedeutung hat, mit Ameisensäure in Gegenwart von Katalysatoren, die zur katalytischen Hydrierung dienen, bei Temperaturen zwischen 0 und 150°C umsetzt.

**Claim**

Process for the preparation of 2-formyl-3,3-dimethyl-cyclopropane-1-carboxylic acid esters of the formula I

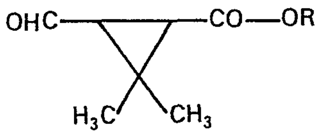

(I)

in which R represents alkyl or optionally substituted aralkyl, characterised in that 2-cyano-3,3-dimethyl-cyclopropane-1-carboxylic acid esters of the formula II

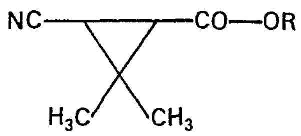

(II)

in which R has the meaning indicated above, are reacted with formic acid in the presence of catalysts which are used for catalytic hydrogenation, at temperatures between 0 and 150°C.

**Revendication**

Procédé pour la préparation d'esters d'acide 2-formyl-3,3-diméthylcyclopropane-1-carboxylique de formule I

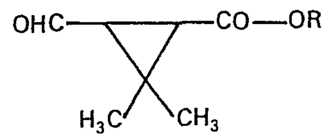

dans laquelle R représente un alkyle ou un arylalkyle éventuellement substitué, caractérisé en ce que l'on fait réagir des esters d'acide 2-cyano-3,3-diméthylcyclopropane-1-carboxylique de formule II

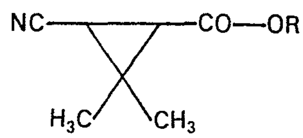

dans laquelle R a la signification indiquée ci-dessus, avec l'acide formique en présence de catalyseurs qui servent à l'hydrogénation catalytique, à des températures entre 0 et 150°C.